# EUROPEAN PATENT SPECIFICATION

(11) **EP 1 003 776 B1**
(45) Date of publication and mention of the grant of the patent: **11.08.2004**
(21) Application number: 98924724.2
(22) Date of filing: 26.05.1998
(51) Int. Cl.: C07K 14/435, C07K 14/47, A61K 38/17

(54) **Screening method using FREAC-11 transcription factor**
Screening-Methode unter Verwendung des FREAC-11 Transkriptionsfaktors
Méthode de criblage utilisant le facteur de transcription FREAC-11

(30) Priority: 26.05.1997 SE 9701963
(43) Date of publication of application: 31.05.2000
(73) Proprietor: BIOVITRUM AB, 112 76 Stockholm (SE)
(72) Inventor: ENERBÄCK, Sven, S-431 96 Mölndal (SE); CARLSSON, Peter, S-448 37 Floda (SE)
(74) Representative: Höglund, Lars
(86) International application number: PCT/SE1998/000989
(87) International publication number: WO 1998/054216

(56) References cited:
- WO-A-95/30026
- GENOMICS, Volume 41, 1997, NAOYUKI MIURA et al., "Isolation of the Mouse (MFH-1) and Human (FKHL14) Mesenchyme Fork Head-1 Genes Reveals Conservation of Their Gene and Protein Structures", pages 489-492.
- FEBS LETTERS, Volume 326, No. 1,2,3, July 1993, NAOYUKI MIURA et al., "MFH-1, A New Member of the Fork Head Domain Family, is Expressed in Developing Mesenchyme", pages 171-176.
- MOLECULAR AND CELLULAR BIOLOGY, Volume 12, No. 10, October 1992, SVEN ENERBACK et al., "Characterization of the Human Lipoprotein Lipase (LPL) Promoter: Evidence of Two Cis-Regulatory Regions, LP-alpha and LP-beta, of Importance for the Differentiation-Linked Induction of the LPLGene During", pages 4622-4633.
- M/S SYNTHESE MEDECINE/SCIENCES, Volume 12, 1996, GENEVIEVE MARTIN et al., "Transcription Differenciation Adipocytaire et Obesite", pages 885-890.

## Description

### Technical field

The present invention relates to a transcription factor. More precisely, the present invention relates to a transcription factor expressed in adipose tissue and involved in lipid metabolism and/or adipocyte differentiation.

### Background of the invention

Triglycerids make up approximately 90% of the dietary fat consumed by an adult on a typical western diet, which is equal to 60-100 gr (Hopfer, 1986). These triglycerids will be processed in a series of enzymatic reactions to release free fatty acids (FFA). FFA will be used as an energy source in muscle tissue or stored as an energy buffer in adipose tissue. The rate limiting step in processing dietary triglycerids to FFA is catalyzed by lipoprotein lipase (LPL) (Brunzell, 1989). Thus, LPL is a key enzyme in providing adipose tissue with FFA, which are reesterified and stored as lipid droplets within the adipocyte. It is therefore reasonable to assume that LPL is critical for developing obesity, a notion underscored by the fact that patients with hyperlipoproteinemia type I (ie. patients with no LPL activity) are typically lean (Holt, 1939).

Furthermore, it is a well established fact that obesity in humans is associated with several diseases such as ischemic heart disease (IHD), noninsulin dependent diabetes mellitus (NIDDM), certain types of cancer and mental disorders.

Substances/methods to decrease fat cell mass in obese individuals with NIDDM are highly desired to restore insulin sensitivity and normalize blood glucose levels.

Obesity is a gradual process in which fat cell size and number increase over time if a person maintains a positive energy balance. A substance that would regulate the rate by which new fat cells are recruited would clearly be of value in treating obesity. One way to achieve this would be to control adipocyte derived lipoprotein lipase or in other ways prevent fat cells from accumulating fat. One such substance is the hormone TNF-α, TNF-α is secreted from several cell types among them adipocytes. It is known that LPL expression is lowered as a consequence of TNF-α administration and that TNF-α might help limiting obesity in some individuals by increasing insulin sensitivity and decreasing LPL (Kern et al, 1995). The effects of endogenous TNF-α on obesity is however of a modest magnitude and to use exogenous TNF-α as a teatment for obesity is most likely of very limited use since the cytokine TNF-α has a wide range of biological activities, eg TNF-α is produced by some tumors and can induce, cacexia ie a pathological vasting syndrome associated with tumors (Oliff, 1988).

The forkhead family of transcription factors (Weigel et al., 1989) is characterized by a conserved (conserved means in this context conserved by evolution giving rise to similar not identical DNA-binding regions among forkhead genes) DNA binding region of some 110 amino acids (Kaufmann et al., 1996). Regions outside of the DNA binding region are not conserved.

Seven so called freac genes are have been reported (Pierrou et al., 1994) belonging to this forkhead family. No one of these freac genes are expressed in adipose tissue.

It is known that forkhead proteins bind to two cis elements in the lpl (lipoprotein lipase) promoter to thereby regulate transcription (Enerbäck, 1992). In the lpl gene these cis elements are designated lp-α (-702 to -666) and 1p-β (- 468 to -430), respectively. These cis elements, show a striking similarity to a concensus sequence known to bind the transcription factors of the HNF 3/ forkhead family, ie.
T/C, A, T, T, G, A, C/T, T. T, A/T, G/T (Enerbäck. supra)
or A/G, T, A, A, A, C/T, A (Pierrou, supra).

Another known gene involved in lipid metabolism is the ob gene (obese) gene ( Da-Wei Gong et al., 1996). In GenBank entry U43589 the sequence of the ob promoter has been deposited.

Further genes of great importance to regulate adipocyte metabolism and/or differentiation are the peroxisome proliferator-activated receptor genes, such as the ppar gamma 2 gene ( Zhu et al., 1995).

### Summary of the invention

The present invention relates to methods for screening for substances influencing the activity of a polypeptide, said polypeptide being a product of a gene that is selectively expressed in adipose tissue stromal cells, adipocytes and preadipocytes. The expression product of the gene has unique ability to regulate genes involved in lipid metabolism and/or adipocyte differentation, such as the lpl gene, and the ob gene. In the present description and claims, genes are designated with lower case letters and gene products are designated with capital letters.

The present invention provides the use of a human transcription factor, called FREAC 11, which is selectively expressed in adipose tissue, as a target in screening. FREAC 11 is a member of the forkhead family of transcription factors (Weigel et al., supra).

For the purposes of the invention, it is essential that the conserved DNA binding domain of FREAC 11 is present, although activities of importance for adipocyte metabolism and/or adipocyte differentiation are expected to reside outside of the 110 amino acid DNA binding region. In FREAC 11 such sites are, for example, sites for protein protein interaction and transcription regulative functions. In freac 11 it is essential that the nucleotide sequence encoding the DNA binding domain is present.

In the methods according to the invention FREAC 11 is used for transcriptional regulation of adipocyte expressed genes selected from the group consisting of the lpl gene, and the ob gene. The present inventor has found that these genes contain potential binding sites for FREAC 11, i.e. at least one cis element in their respective promoter. The number of cis elements is two for the lpl gene, three for the ob gene, and three for the ppar gamma 2 gene, respectively.

The 110 amino acid DNA binding domain of FREAC 11 binds to these cis elements. For the skilled man in the art it is evident that other preadipocyte and adipocyte expressed genes are very likely to be regulated as well if these cis elements are present.

The invention is defined by the attached claims.

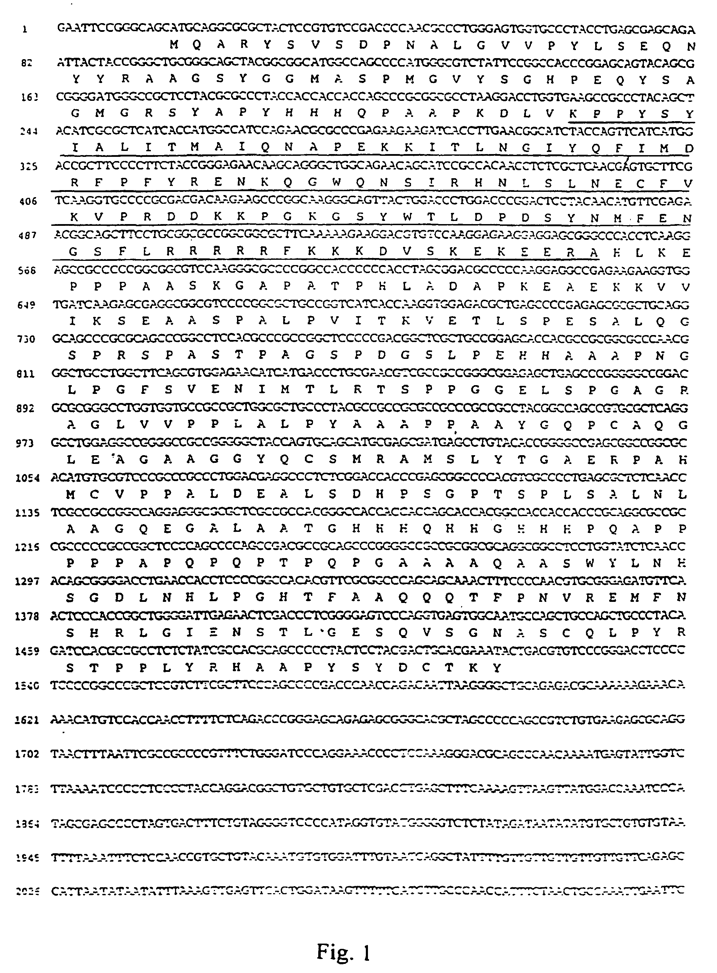

The invention relates to a method for screening for substances influencing the activity of a FREAC 11 transcription factor comprising the amino acid sequence of Fig. 1, said method comprising determining whether a substance influences the binding of said factor to a gene involved in lipid metabolism, said gene selected from the group consisting of the lpl gene and the ob gene.

For the purpose of the invention, gene means both genomic DNA, cDNA, and synthetic DNA.

The substances identified by the method of the invention are intended for use in therapy of obesity-related conditions where it is desired to either increase or decrease the activity of adipocyte expressed genes, such as obesity, non-insulin dependent diabetes mellitus, cardiovascular diseases, catabolic conditions, anorexia, bulemia.

### Detailed description of the invention

The invention will now be described more closely below in association with the accompanying drawings and some non-limiting examples.

In the drawings:
Fig. 1 shows nucleotide and amino acid sequence of FREAC 11 wherein the DNA binding domain is underlined;
Fig. 2 shows a Northern blot of RNA from different tissues using a part of the cDNA in fig 1 located outside of the DNA-binding region as a probe;
Fig. 3 shows a diagram of the induction of LPL during adipogenesis measured as reporter gene activity derived from a lpl promoter-reporter gene construct; the filled bars show 3T3-F442A cells transfected with a lpl promoter-reporter gene construct only; and the open bars show a corresponding experiment with a lpl promoter- reporter gene construct that has been transfected into differentiating 3T3-F442A adipocytes together with a construct expressing only the DNA binding region of freac 11.
Fig. 4 shows a diagram of the induction of OB during adipogenesis measured as reporter gene activity derived from a ob promoter-reporter gene construct; the left hand bar shows CHO cells transfected with a ob promoter-reporter gene construct only; and the right hand bar shows a corresponding experiment with a ob promoter-reporter gene construct that has been transfected together with a construct expressing only the DNA binding region of freac 11.
Fig 5 shows body weight of transgenic mice having extra copies of FREAK 11 and control.

### EXAMPLE 1: FREAC 11 is expressed selectively in adipose tissue

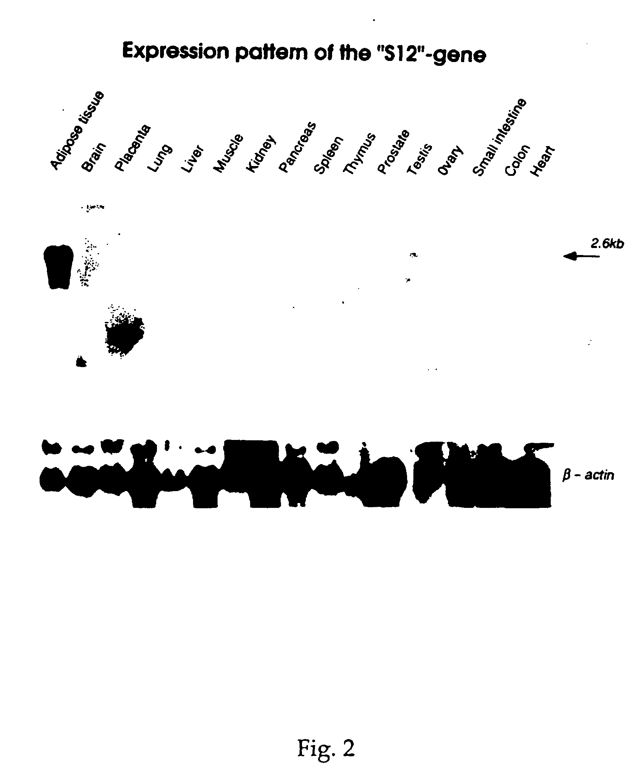

mRNA from sixteen different tissues were examined by Northern blotting using a cDNA probe derived from a region outside of the DNA binding region freac- 11 (fig 1). The 16 different tissues were adipose tissue, brain, placenta, lung, liver, muscle, kidney, pancreas, spleen, thymus, prostate, testis, ovary, small intestine, colon, heart. The Northern blotting shown in Fig. 2 with RNA from the above mentioned 16 different tissues demonstrates that only in RNA derived from adipose tissue the freac 11 transcript is clearly detected.

### EXAMPLE 2: Transfected freac 11 inhibits LPL promoter activity

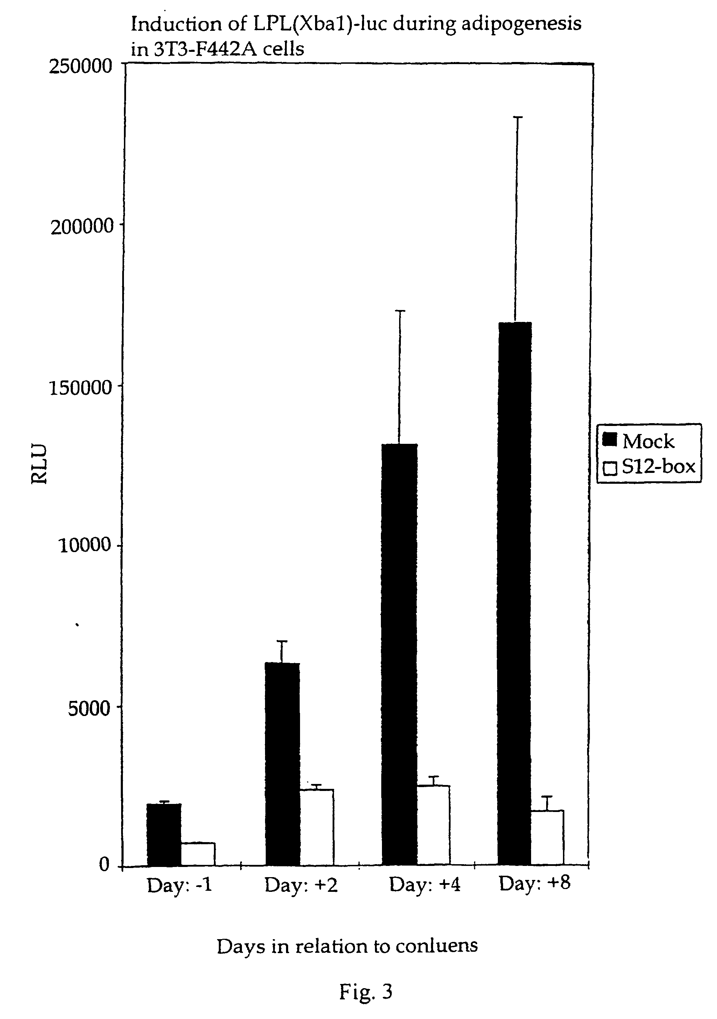

To study the regulative properties of FREAC 11 a co-transfection was performed in a way that mimics the endogeneous lpl mRNA level in 3T3-F442A cells as depicted in Fig. 3.

The reporter gene in this Example comprises a fragment of the human lpl promoter sequence described by Enerbäck et al.. supra. This fragment is positioned 54 nucleotides upstream of the translation start of the lpl gene reaching 989 nucleotides upstream into the lpl promoter sequence harboring both forkhead binding sites. To construct the reporter gene, this fragment was fused to a luciferase reporter gene. The cells were harvested at various time points, day-1 indicating the day before confluence of growth and day +2. +4 and +8 indicating days of culture after confluence.

Fig. 3 shows that when an expression vector encoding the DNA binding region (= S12 box) of freac 11 is transfected together with a lpl promoter- reporter gene, the normal step wise induction of LPL during adipogenesis is inhibited whereas no such inhibition is present in the closed bar experiment, see legend to Fig. 3. Expression of S12 box (open bars) inhibits the normal LPL induction (filled bars).

In this way it is possible to study the effects of the endogenously expressed FREAC-11 in these cells by blocking its access to the lpl promoter.

The transcription factor FREAC 11 according to the invention is exclusively expressed in adipose tissue when the DNA binding domain of this transcription factor is expressed in stable transfectants. It will act as a negative dominant mutation and LPL levels, measured as reporter gene activity, are down to base line values, see Fig. 3. Thus, the DNA binding domain of FREAC 11 will bind to the two cis elements in the LPL promoter- in doing so this cis elements will be blocked and not able to interact with wild type transcription factors.

### EXAMPLE 3: Transfected freac 11 inhibits ob promoter activity

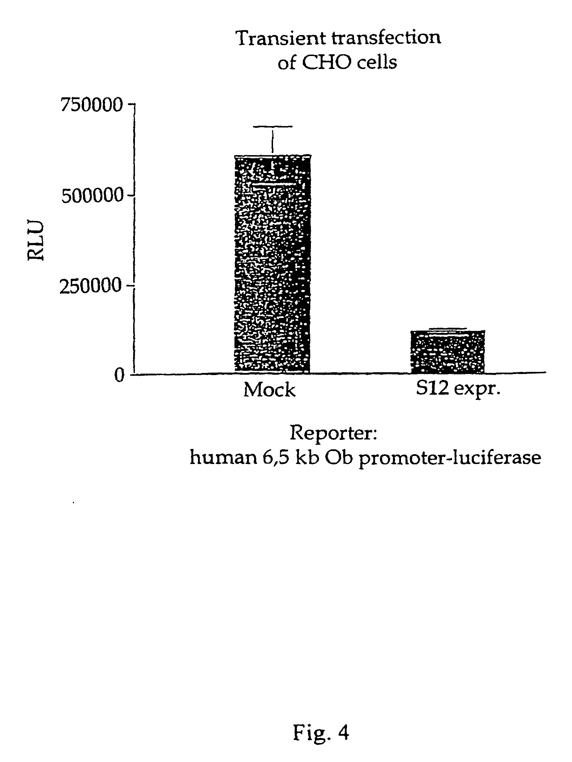

To study the regulative properties of FREAC 11 a co-transfection was performed as depicted in Fig. 4. This shows that when an expression vector encoding the DNA binding region (= S12 box) of freac 11 is transfected together with a ob promoter-reporter gene a clear reduction in reporter gene activity is evident. This implicates FREAC- 11 not only as a regulator of lpl gene but also as a regulator of the ob gene. This experiment provides evidence that the ob promoter contains functional cis-elements capable of interacting with the DNA binding domain of FREAC 11. Both freac 11 and the ob-gene are selectively expressed in adipose tissue.

### EXAMPLE 4.Body weight of trangenic mice

The body weight of control. transgenic mice with 1-3 extra copies of FREAC 11 (low copy transgenic) in the genom and transgenic mice having more than five extra copies of FREAC 11 in the genom 11 (high copy transgenic) was compared in 73 animals.
It was found that the body weight is significantly elevated in high copy transgenic mice in comparison to low copy transgenic and control, both for male and female.

The figures found were:

| | Mean Diff. | Crit. Diff | P-value |
|---|---|---|---|
| Controls vs Low copy transgenic | -1.475 | 2.899 | 0.1817 |
| Controls vs High copy transgenic | -6.725 | 4.366 | 0.0001 S |
| Low copy vs High copy transgenic | -5.250 | 4.842 | 0.0054 S |
| S is significant | | | |

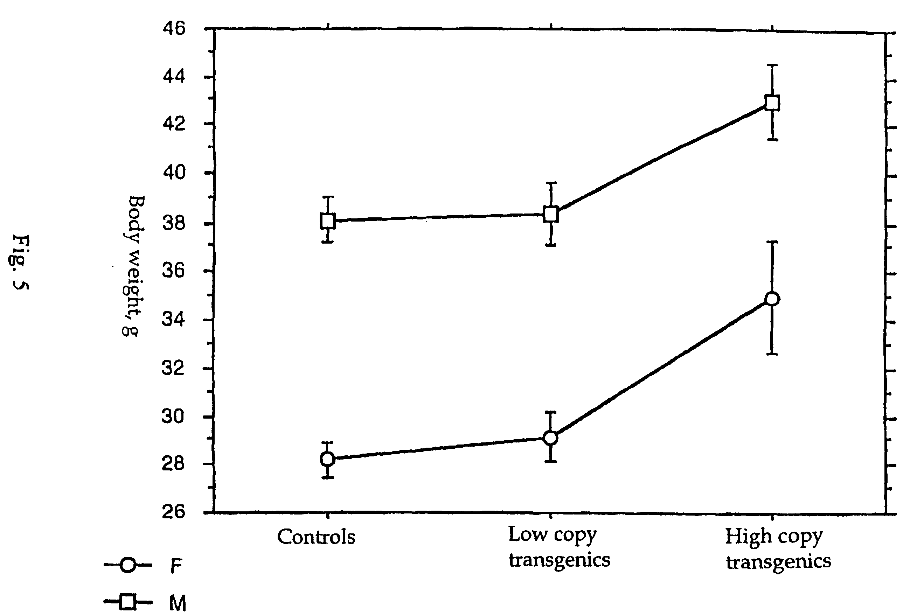

In Figure 5 Body Weight in gram is given for controls, low copy transgenic and high copy transgenic, respectively.
Significance level: 1%
Error Bars: ±1 Standard Error(s)

### EXAMPLE 5: FREAC-11 for high throughput screening

### a) Substances influencing the DNA binding of FREAC-11

For this purpose, FREAC 11 shall be expressed and used in gel-mobility-shift experiments to evaluate DNA binding specificity, such an assay could be used to screen substances that are capable of inhibiting and/or in other ways interfere or modulate the way in which FREAC-1 interacts with DNA.

### b) Antagonists/Agonists of FREAC-11

Here substances/molecules will be screened to obtain those having ability to influence function of FREAC 11 as a transcription factor, such as agonists and antagonists. As demonstrated in figure 3, a dominant negative mutation of FREAC 11, only containing the DNA binding region can when the corresponding gene is cotransfected abolish the differentiation linked induction of LPL in the adipocyte differentiating cell line 3T3-F442A. In further experiments, various derivatives of FREAC 11 will be used to investigate other biological effects of FREAC 11.

### c) Substances influencing the interaction of FREAC-11 with other proteins/molecules

Through the use of yeast 2- hybrid screening systems, or similar systems, possible protein - protein interactions will be investigated - such proteins ie proteins that interact with FREAC-11 could also be used to modulate the activity of FREAC-11 and in doing so also regulating LPL and adipocyte differentiation /metabolism.

Besides proteins, the interaction of FREAC 11 with other molecules than proteins, such as carbohydrats, lipids, and other compounds. will also be investigated.

### EXAMPLE 6. The freac-11 gene as a drug target

For this purpose an antisense construct of freac 11 will be produced. If freac 11 is inhibited in this way, an increased activity is expected of adipocyte expressed genes containing the described cis elements.

### References

Hopfer, U. (1986) *Biochemistry* (Devlin, T. ed. second edition), 931-938. John Wiley & Sons. New York.
Brunzell, J.D. (1989) *The Metabolic Basis of Inherited Disease* (Scriver. C.R.. Beaudet, A.L. and Valle, D. eds.), 1165-1180. McGraw-Hill, New York.
Holt, L.E., Jr.; Aylward, F.X.; Timbers, H.G.: Idiopathic familial lipemia. *Bull. Johns Hopkin Hosp.* 64; 279-314.
Kern P.A., (1995) The expression of tumor necrosis factor in human adipose tissue. Regulation by obesity, weight loss, and relationship to lipoprotein lipase in *J Clin Invest 5;* 2111-2119.
Oliff, A., (1988). The role of tumor necrosis factor (cachectin) in cachexia. Cell 15;54(2):141-2.
Weigel, D., Jurgens, G., Kuttner, F., Seifert, E., and Jackle, H. (1989). The homeotic gene fork head encodes a nuclear protein and is expressed in the terminal regions of the Drosophila embryo. *Cell 57:645-58.*
Kaufmann, E., and Knochel, W. (1996). Five years on the wings of fork head. *Mech Dev* 57:3-20.
Pierrou, S., Hellqvist, M., Samuelsson, L., Enerbäck. S.. and Carlsson, P. (1994). Cloning and characerization of seven human forkhead proteins: binding site specificity and DNA binding. *Embo J* 1354:5002-5012.
Da-Wei Gong, Sheng Bi, Richard E. Pratley and Bruce D. Weintraub. (1996) Genomic structure and promoter analysis of the human obese gene. *J Biol Chem* 271(8):3971-3974.
Zhu Y., Qi C., Korenberg J. R., Chen X. N., Noya D.. Rao M.S. and Reddy J.K. Structural organization of mouse peroxisome proliferator-activated receptor gamma (mPPAR gamma) gene; alternative promoter use and different splicing yield two mPPAR gamma isoforms. *Proc. Natl Sci USA* 92: 7921-7925.
Enerbäck, S., Ohlsson, B. G., Samuelsson, L., and Bjursell, G. (1992). Characterization of the human lipoprotein lipase (LPL) promoter: evidence of two cis-regulatory regions. LP-alpha and LP-beta, of importance for the differentiation-linked induction of the LPL gene during adipogenesis. *Mol Cell Biol* 12: 4622-33.

## Claims

1. A method for screening for substances influencing the activity of a polypeptide comprising the amino acid sequence shown in Figure 1, said method comprising determining whether a substance influences the binding of the said polypeptide to a gene involved in lipid metabolism, said gene selected from the group consisting of the lpl gene and the ob gene.

2. The method according to claim 1 wherein the said gene involved in lipid metabolism is the lpl gene.

3. The method according to claim 1 wherein the said gene involved in lipid metabolism is the ob gene.

4. The method according to any one of claims 1 to 3 wherein the said polypeptide consists of the amino acid sequence shown in Figure 1.

5. The method according to any one of claims 1 to 4 for identifying a substance useful for the treatment of an obesity-related condition.

6. The method according to claim 5 wherein the said obesity-related condition is obesity, non-insulin dependent diabetes mellitus, cardiovascular diseases, catabolic conditions, anorexia or bulimia.

## Patentansprüche

1. Ein Screening-Verfahren für Substanzen, die die Aktivität eines Polypeptids, das die in Figur 1 dargestellte Aminosäuresequenz umfasst, beeinflussen, wobei das Verfahren umfasst, zu bestimmen, ob eine Substanz die Bindung dieses Polypeptids an ein Gen, das am Lipidmetabolismus beteiligt ist, beeinflusst, wobei das Gen aus der Gruppe ausgewählt ist, die aus dem lpl-Gen und dem ob-Gen besteht.

2. Das Verfahren gemäß Anspruch 1, worin das Gen, das am Lipidmetabolismus beteiligt ist, das lpl-Gen ist.

3. Das Verfahren gemäß Anspruch 1, worin das Gen, das am Lipidmetabolismus beteiligt ist, das ob-Gen ist.

4. Das Verfahren gemäß einem der Ansprüche 1 bis 3, worin das Polypeptid aus der in Figur 1 dargestellten Aminosäuresequenz besteht.

5. Das Verfahren gemäß einem der Ansprüche 1 bis 4 zur Identifizierung einer Substanz, die zur Behandlung eines Fettsucht-bezogenen Zustands geeignet ist.

6. Das Verfahren gemäß Anspruch 5, worin der Fettsucht-bezogene Zustand Fettsucht, insulinunabhängiger Diabetes mellitus, Herz-Kreislauf-Erkrankungen, katabolische Zustände, Anorexie oder Bulimie ist.

## Revendications

1. Procédé de criblage de substances influençant l'activité d'un polypeptide comprenant la séquence d'acides aminés représentée à la Figure 1, ledit procédé comprenant : déterminer si une substance influence la liaison dudit polypeptide avec un gène impliqué dans le métabolisme des lipides, ledit gène choisi dans le groupe consistant en le gène lpl et le gène ob.

2. Procédé selon la revendication 1, dans lequel ledit gène impliqué dans le métabolisme des lipides est le gène lpl.

3. Procédé selon la revendication 1, dans lequel ledit gène impliqué dans le métabolisme des lipides est le gène ob.

4. Procédé selon l'une quelconque des revendications 1 à 3, dans lequel ledit polypeptide consiste en la séquence d'acides aminés représentée à la Figure 1.

5. Procédé selon l'une quelconque des revendications 1 à 4 pour identifier une substance utile pour le traitement d'un état associé à l'obésité.

6. Procédé selon la revendication 5, dans lequel ledit état associé à l'obésité est l'obésité, le diabète sucré non insulino-dépendant, les maladies cardiovasculaires, les états cataboliques, l'anorexie ou la boulimie.
